# EUROPEAN PATENT SPECIFICATION

(11) **EP 0 949 255 B1**
(45) Date of publication and mention of the grant of the patent: **24.05.2006**
(21) Application number: 99106507.9
(22) Date of filing: 30.03.1999
(51) Int. Cl.: C07D 311/72

(54) **Process for manufacturing d, l-alpha-tocopherol in a carbonate solvent and in the presence of a sulphur-containing acid catalyst**
Verfahren zur Herstellung von d,l-alpha-Tocopherol in einem Carbonat-Lösungsmittel und in Anwesenheit eines sauren schwefelhaltigen Katalysators
Procédé de préparation de d,l-alpha-tocophérol utilisant comme solvant un carbonate et comme catalyseur un acide contenant du soufre

(30) Priority: 06.04.1998 EP 98106237
(43) Date of publication of application: 13.10.1999
(73) Proprietor: DSM IP Assets B.V., 6411 TE Heerlen (NL)
(72) Inventor: Baak, Marcel, 3232 Ins (CH); Bonrath, Werner, 79115 Freiburg (DE); Kreienbuehl, Paul, 4125 Riehen (CH)
(74) Representative: Müller, Ingrid

(56) References cited:
- EP-A- 0 694 541
- WO-A-97/28151
- US-A- 3 708 505
- KAJIWARA M. ET AL.: "The synthesis of regiospecifically 13C-labeled .alpha.-tocopherol acetate" HETEROCYCLES, vol. 15, no. 2, 1981, pages 1209-1212, XP002110587
- URANO S. & MATSUO M.: "The synthesis of C-13 labeled vitamin E, [2a-13C]all-rac-.alpha.-tocopherol" HETEROCYCLES, vol. 22, no. 9, 1984, pages 1975-1977, XP002110588

## Description

The present invention is concerned with a novel process for the manufacture of d,l-α-tocopherol by the acid-catalyzed condensation of trimethylhydroquinone (TMHQ) with isophytol (IP) in a solvent. As is known, d,l-α-tocopherol is a diastereoisomeric mixture of 2,5,7,8-tetramethyl-2-(4',8',12'-trimethyl-tridecyl)-6-chromanol (α-tocopherol), which is the most active and industrially most important member of the vitamin E group.

Many processes for the manufacture of d,l-α-tocopherol by the condensation of TMHQ with IP in the presence of various catalysts or catalyst systems and in various solvents are described in the literature. These processes go back to the work of Karrer et al., Bergel et al. as well as Smith et al. [see Helv. Chim. Acta 21, 520 et seq. (1938), Nature 142, 36 et seq. (1938) and, respectively, Science 88, 37 et seq. (1938) and J. Am. Chem. Soc. 61, 2615 et seq. (1939)]. While Karrer et al. carried out the synthesis of d,l-α-tocopherol from TMHQ and phytyl bromide in the presence of anhydrous zinc chloride (ZnCl₂; a Lewis acid), not only Bergel et al. but also Smith et al. used TMHQ and phytol as starting materials. In the following years mainly modifications, e.g. alternative solvents and Lewis acids, were developed. From the work of Karrer et al. there was developed in the year 1941 a technically interesting process for the manufacture of d,l-α-tocopherol which was based on the condensation of TMHQ with IP in the presence of the catalyst system Zncl₂/hydrochloric acid (HCl) (US Patent 2 411 969). Later publications, e.g. Japanese Patent Publications (Kokai) 54380/1985, 64977/1985 and 226979/1987 [Chemical Abstracts (C.A.) 103, 123731s (1985), C.A. 103, 104799d (1985) and, respectively, C.A. 110, 39217r (1989)], describe this condensation in the presence of zinc and ZnCl₂ and a Bronsted (protonic) acid, such as a hydrohalic acid, e.g. HCl, trichloroacetic acid, acetic acid and the like, especially ZnCl₂/HCl, as the catalyst system. Disadvantages of these and further published processes featuring ZnCl₂ in combination with a Bronsted acid are the corrosive properties of the acids and the contamination of the waste water with zinc ions as a result of the large amount of ZnCl₂ required for the catalysis.

The manufacture of d,l-α-tocopherol by the reaction of TMHQ with phytyl chloride, phytol or isophytol in the presence of boron trifluoride (BF₃) or its etherate (BF₃·Et₂O) is described in German Patents 960720 and 1015446 as well as in US Patents 3 444 213 and 4 634 781. However BF₃ too has corrosive properties.

Also, the condensation of TMHQ with IP or another phytyl derivative in the presence of a Lewis acid, e.g. ZnCl₂, BF₃ or aluminium trichloride (AlCl₃), a strong acid, e.g. HCl, and an amine salt as the catalyst system is described in European Patent Publication (EP) 100471. In an earlier patent publication, DOS 2606830, the IP or phytol is pretreated with ammonia or an amine before the condensation with TMHQ in the presence of ZnCl₂ and an acid is effected. In both cases corrosion problems occur.

A further interesting method for the manufacture of d,l-α-tocopherol from TMHQ and IP comprises using an isolated TMHQ-BF₃ or -AlCl₃ complex as the catalyst and a solvent mixture featuring a nitro compound (DOS 1909164). This process avoids to a large extent the formation of undesired by-products because it involves mild reaction conditions. The yield of d,l-α-tocopherol, based on IP and the use of the solvent mixture methylene chloride/nitromethane, is given as 77%. However, the use of such a solvent mixture is disadvantageous.

The manufacture of d,l-α-tocopherol by the condensation of TMHQ with IP using cation exchange complexes of metal ions (Zn²⁺, Sn²⁺ and Sn⁴⁺) is disclosed in Bull. Chem. Soc. Japan 50, 2477-2478 (1977); amongst other disadvantages it gives the desired product in unsatisfactory yields.

The use of macroreticular ion exchangers, e.g. Amberlyst® 15 as the catalyst for the condensation of TMHQ with IP is described in US Patent 3459773.

EP 603695 describes the manufacture of d,l-α-tocopherol in liquid or supercritical carbon dioxide by the condensation of TMHQ with IP in the presence of acidic catalysts, such as ZnCl₂/HCl and ion exchangers.

The condensation in the presence of a catalyst system which consists of iron(II) chloride, metallic iron and HCl gas is described in DOS 2160103 and US Patent 3789086. The formation of less byproducts is advantageous compared with the aforementioned process using ZnCl₂/HCl. However, corrosion problems and chloride contamination are equally disadvantageous.

An interesting alternative for the condensation of TMHQ with IP to d,l-α-tocopherol comprises using trifluoroacetic acid or its anhydride as the catalyst (EP 12824). Although in this process the avoidance of HCl ist achieved, the alternative catalyst is relatively expensive.

The use of heteropolytungsten acids as catalysts for the condensation of TMHQ with IP was described for the first time in React. Kinet. Catal. Lett. 47(1), 59-64 (1992). d,l-α-Tocopherol could be obtained in about 90% yield with this process using various solvents.

A further process described in the literature [EP 658552; Bull. Chem. Soc. Japan 68, 3569-3571 (1995)] for the synthesis of d,l-α-tocopherol is based on the use of a scandium, yttrium or lanthanide fluorosulphonate, nitrate or sulphate, e.g. scandium trifluoromethanesulphonate. With up to about 10% excess of IP this process gives yields up to 98%.

The use of ion exchanged bentonite, montmorillonite or saponite through treatment with e.g. scandium chloride and other metal salts (yttrium, lanthanum, etc.) as the catalyst for the condensation of TMHQ with IP has as a disadvantage the need for a large amount of catalyst [EP 677520; Bull. Chem. Soc. Japan 69, 137-139 (1996)].

According to the Examples of EP 694 541 the condensation of TMHQ with IP to α-tocopherol can be achieved in high yields and with a high product purity when such solvents as carbonate esters, fatty acid esters and mixed solvent systems are employed, catalysis being effected by ZnCl₂/HCl. Disadvantages in this process are, in addition to the contamination of the waste water by zinc ions, the usual large "catalyst amount" of ZnCl₂ used.

According to WO 97/28151 the acid-catalysed condensation of TMHQ with IP can be performed in a cyclic carbonate or α-lactone as the solvent. The preferred catalyst is a mixture of ortho boric acid and oxalic, tartaric or citric acid, or boron trifluoride etherate.

From the forgoing explanations it is evident that most of the previously known processes have considerable disadvantages. Thus, corrosion problems occur in all processes in which such acid catalysts as boron trifluoride are used. Toxicity problems with the boron trifluoride adducts also occur, and when iron or zinc is used there is a contamination of the waste water with the metal ions which is today no longer acceptable. In some processes the formation of undesired byproducts, e.g. phytyltoluene and chlorophytols, is an especially serious problem.

The object of the present invention is to provide a process for the manufacture of d,l-α-tocopherol by the condensation of trimethylhydroquinone with isophytol in the presence of a catalyst and in a solvent which does not have the disadvantages of previously known procedures. In this respect, it is necessary that the catalyst used has no, or at least a much reduced, corrosive action, is non-toxic, does not contaminate the environment and catalyzes the desired reaction as selectively as possible and in high yields. Furthermore, the catalyst should display its activity already in small, really catalytic, amounts, and should be readily separable and re-usable several times.

This object of the present invention is achieved by carrying out the condensation of trimethylhydroquinone with isophytol in the presence of at most 0.4 weight percent based on the weight of isophytol of one of the following sulphur-containing acid catalysts: sulphuric acid, methanesulphonic acid, ethanesulphonic acid, trifluoromethanesulphonic acid, p-toluenesulphonic acid and fluorosulphonic acid, so rendering the use of a Lewis acid unnecessary. Furthermore, condensation is effected in ethylene or propylene carbonate or a mixture of both carbonates, or in a mixture of one or both of the carbonates and a non-polar solvent as the solvent or (two-phase) solvent system, as appropriate.

The condensation itself is represented in the following Reaction Scheme, which is presented conventionally:

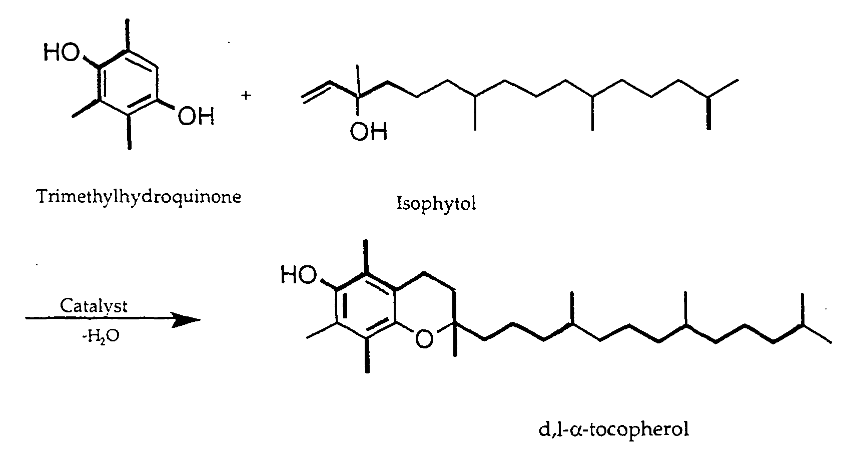

Thus, the process in accordance with the invention for the manufacture of d,l-α-tocopherol by the acid-catalyzed condensation of trimethylhydroquinone with isophytol in ethylene or propylene carbonate or a mixture of both carbonates, or in a mixture of one or both of the carbonates and a non-polar solvent, is characterized by carrying out the condensation in the presence of at most 0.4 weight percent based on the weight of isophytol of an acid catalyst selected from sulphuric acid, methanesulphonic acid, ethanesulphonic acid, trifluoromethanesulphonic acid, p-toluene sulphonic acid and fluorosulphonic acid.

If in addition to ethylene or propylene carbonate or a mixture of both carbonates and a non-polar solvent is employed, said non-polar solvent is suitably an aliphatic hydrocarbon, e.g. hexane, heptane or petroleum ether, preferably heptane.

The condensation is conveniently effected at temperatures from about 50°C to about 150°C, preferably from about 80°C to about 120°C, especially at about 100°C.

Furthermore, trimethylhydroquinone is conveniently used in a molar excess over isophytol of about 30 to 65%, preferably about 50%. The amount of acid catalyst is conveniently about 0.1 to 0.4 weight percent, preferably about 0.1 to 0.2 weight percent, based on the weight of isophytol.

If the reaction is carried out in the presence of both a carbonate (ethylene or propylene carbonate, or both) and a non-polar solvent, then the volume ratio of the non-polar solvent to the carbonate used in the two-phase solvent system is conveniently in the range from 0.3:1 to 5:1. The (total) amount of carbonate solvent, i.e. ethylene carbonate and/or propylene carbonate is conveniently about 10 to 100 ml, preferably about 10 to 50 ml, per 100 mmol of trimethylhydroquinone, and if a non-polar solvent is additionally used, conveniently about 50 to 150 ml, preferably about 70 to 120 ml, of said non-polar solvent are used per 100 mmol of isophytol. In any event, only the one or the other carbonate is preferably used, either as the sole solvent or as the carbonate component of the two-phase solvent system with the non-polar solvent. As the latter, heptane is preferably employed, and the carbonate itself is preferably ethylene carbonate.

Moreover, the condensation is conveniently carried out under an inert gas atmosphere, preferably gaseous nitrogen or argon.

As the acid catalyst there is preferably used sulphuric acid or p-toluenesulphonic acid.

The process in accordance with the invention can be carried out operationally in a very simple manner by adding isophytol alone or a solution of isophytol in the optionally employed non-polar solvent dropwise to a solution or suspension of the trimethylhydroquinone and the acid catalyst in ethylene or propylene carbonate or a mixture of both carbonates. The rate at which the isophytol is added is not critical. Conveniently, isophytol is added dropwise over a period of 0.1 to 1, preferably 0.3 to 0.5, hour. After completion of the isophytol addition and an appropriate subsequent condensation, during which it is advantageous to remove the resulting water by azeotropic distillation or in the flow of inert gas used, isolation and purification of the obtained d,l-α-tocopherol can be effected by procedures conventionally used in organic chemistry, e.g. by distillation.

Particular advantages in the use of the acid catalyst in the process in accordance with the invention are, in addition to high yields of d,l-α-tocopherol, the avoidance of waste water contamination with heavy metal ions, the high selectivity as well as the enabled ready isolation of the produced d,l-α-tocopherol from the mixture after reaction, in particular from the unreacted trimethylhydroquinone.

The process in accordance with the invention is illustrated by the following Examples:

### Example 1

To a mixture of 23.3 g (150 mmol) of 2,3,5-trimethylhydroquinone (98% pure), 80 ml of ethylene carbonate (99% pure) and 0.14 ml of 10% (v/v) sulphuric acid (0.25 mmol), a solution of 31.21 g (100 mmol) of isophytol (95% pure) in 100 ml of heptane was added dropwise under an argon atmosphere and with stirring at 100°C over a period of 20 minutes. During the addition of isophytol an azeotropic mixture of water/heptane was separated with the help of a water separator. After completion of the addition, the reaction mixture was stirred for another 30 minutes with removal of heptane. The internal temperature rose to about 145°C. After cooling the reaction mixture to about 90°C, 200 ml of heptane were added and the mixture was stirred for about 5 minutes. The upper layer was separated and the lower carbonate layer was extracted with a further 100 ml of heptane. The combined heptane layers were washed with 20 ml of water. After removal of heptane by evaporation 43.76 g (95.6% yield) of d,l-α-tocopherol were obtained as a brown oil [purity as determined by gas chromatographic (GC) analysis: 94.1%].

### Example 2

To a mixture of 23.3 g (150 mmol) of 2,3,5-trimethylhydroquinone (98% pure), 80 ml of ethylene carbonate (99% pure) and 0.28 ml of 10% (v/v) sulphuric acid (0.50 mmol), a solution of 31.21 g (100 mmol) of isophytol (95% pure) in 100 ml of heptane was added dropwise under an argon atmosphere with stirring at 100°C over a period of 20 minutes. During the addition of isophytol an azeotropic mixture of water/heptane was separated with the help of a water separator. After completion of the addition, the reaction mixture was stirred for another 30 minutes with removal of heptane. The internal temperature rose to about 145°C. After cooling the reaction mixture to about 90°C, 200 ml of heptane were added and the mixture was stirred for about 5 minutes. The upper layer was separated and the lower carbonate layer was extracted with a further 100 ml of heptane. The combined heptane layers were washed with 20 ml of water. After removal of heptane by evaporation 43.71 g (95.6% yield) of d,l-α-tocopherol were obtained as a brown oil (purity according to GC analysis: 94.2%).

### Example 3

To a mixture of 93.18 g (600 mmol) of 2,3,5-trimethylhydroquinone (98% pure), 80 ml of ethylene carbonate (99% pure) and 1.12 ml of 10% (v/v) sulphuric acid (2.0 mmol), a solution of 124.85 g (400 mmol) of isophytol (95% pure) in 400 ml of heptane was added dropwise under an argon atmosphere with stirring at 100°C over a period of 20 minutes. During the addition of isophytol an azeotropic mixture of water/heptane was separated with the help of a water separator. After completion of the addition, the reaction mixture was stirred for another 30 minutes with removal of heptane. The internal temperature rose to about 145°C. After cooling the reaction mixture to about 90°C, 200 ml of heptane were added and the mixture was stirred for about 5 minutes. The upper layer was separated and the lower carbonate layer was extracted with a further 100 ml of heptane. The combined heptane layers were washed with 20 ml of water. After evaporation of the heptane 174.84 g (95.6% yield) of d,l-α-tocopherol were obtained as a brown oil (purity according to GC analysis: 94.2%).

### Examples 4-9

In a flask with reflux condenser, water collector and mechanical stirrer 23.3 g (150 mmol) of 2,3,5-trimethylhydroquinone (98% pure) and the acid catalyst [p-toluenesulphonic acid (p-TsOH) or another sulphur-containing acid] were dissolved in 80 ml of ethylene carbonate. A solution of 36.55 ml (100 mmol) of isophytol (96% pure) in 100 ml of heptane was added within 30 minutes at about 100°C and the reaction mixture was heated for 30 minutes with removal of heptane. The mixture was then heated at 135°C for 30 minutes. The resulting two-phase system was cooled to 80°C, and 100 ml of heptane were added. The two phases were separated and the lower carbonate layer was re-used. The upper heptane layer was concentrated under reduced pressure. The so-obtained and isolated crude d,l-α-tocopherol was analyzed by GC to determine the purity. The results obtained are summarized in the following Table.

**Table**

| Example | Catalyst (weight % based on IP) | Yield (%) | Purity (%) |
|---|---|---|---|
| 4 | p-TsOH (0.13) | 92.0 | 87.0 |
| 5 | H₂SO₄ (0.25) | 93.3 | 89.6 |
| 6 | C₂H₅SO₃H (0.33) | 91.8 | 88.4 |
| 7 | CH₃SO₃H (0.35) | 95.0 | 90.8 |
| 8 | CF₃SO₃H (0.37) | 95.7 | 89.7 |
| 9 | FSO₃H (0.12) | 92.1 | 86.4 |

### Example 10

In a flask with reflux condenser, water collector and mechanical stirrer 23.3 g (150 mmol) of 2,3,5-trimethylhydroquinone (98% pure) and 0.1 g of p-toluenesulphonic acid were dissolved in 80 ml of propylene carbonate. A solution of 36.55 ml (100 mmol) of isophytol (96% pure) in 100 ml of hexane was added within 30 minutes at about 100°C and the reaction mixture was heated for 30 minutes while removing the hexane. The mixture was then heated at 135°C for 30 minutes. The resulting two-phase system was cooled to 80°C and 100 ml of hexane were added. The two phases were separated and the lower carbonate layer was re-used. The upper hexane layer was concentrated under reduced pressure and yielded 39.75 g (92.3 % yield) of crude d,l-α-tocopherol (purity according to GC analysis: 87.0%).

### Example 11

In a flask with reflux condenser, water collector and mechanical stirrer 23.3 g (150 mmol) of 2,3,5-trimethylhydroquinone (98% pure), and 0.1 g of p-toluenesulphonic acid were dissolved in 80 ml of ethylene carbonate. A solution of 36.55 ml (100 mmol) of isophytol (96%) was added within 30 minutes at about 100°C and the reaction mixture was heated for 30 minutes. The reaction solution was then heated at 135°C for 30 minutes and cooled to 80°C. The two phases were separated; the upper layer consisted of 39.23 g (91.1 % yield) of crude d,l-α-tocopherol (purity according to GC analysis: 87.1%).

## Claims

1. A process for the manufacture of d,l-α-tacopherol by the acid-catalyzed condensation of trimethylhydroquinone with isophytol in ethylene or propylene carbonate or a mixture of both carbonates or in a mixture of one or both of the carbonates and a non-polar solvent, which process is **characterized by** carrying out the condensation without using a Lewis acid, in the presence of at most 0.4 weight percent based on the weight of isophytol of an acid catalyst selected from sulphuric acid, methanesulphonic acid, ethanesulphonic acid, trifluoromethanesulphonic acid, p-toluenesulphonic acid and fluorosulphonic acid.

2. A process according to claim 1, wherein the catalyst is sulphuric acid or p-toluenesulphonic acid.

3. A process according to claim 1 or 2, wherein the amount of acid catalyst is about 0.1 to 0.4, preferably about 0.1 to 0.2 weight percent, based on the weight of isophytol.

4. A process according to any one of claims 1 to 3, wherein the condensation is carried out in ethylene carbonate or a mixture thereof with a non-polar solvent.

5. A process according to any one of claims 1 to 4, wherein the non-polar solvent is hexane, heptane or petroleum ether, preferably heptane.

6. A process according to any one of claims 1 to 5, wherein the volume ratio of the non-polar solvent to the carbonate in the two-phase solvent system is in the range from 0.3:1 to 5:1.

7. A process according to any one of claims 1 to 6, wherein the condensation is effected at temperatures from about 50°C to about 150°C, preferably from about 80°C to about 120°C, especially at about 100°C.

8. A process according to any one of claims 1 to 7, wherein trimethylhydroquinone is used in a molar excess over isophytol of about 30 to 65%, preferably about 50%.

9. A process according to any one of claims 1 to 8, wherein the condensation is carried out under an inert gas atmosphere, preferably gaseous nitrogen or argon.

10. A process according to any one of claims 1 to 9, wherein about 10 to 100 ml, preferably about 10 to 50 ml, of carbonate solvent (ethylene and/or propylene carbonate) are used per 100 mmol of trimethylhydroquinone and, if a non-polar solvent is additionally used, about 50 to 150 ml, preferably about 70 to 120 ml, of non-polar solvent are used per 100 mmol of isophytol.

11. A process according to any one of claims 1 to 10, wherein isophytol or a solution of isophytol in the employed non-polar solvent is added dropwise to a solution or suspension of trimethylhydroquinone and the acid catalyst in a ethylene or propylene carbonate or a mixture of both carbonates.

12. A process according to any one of claims 1 to 11, wherein the water resulting in the condensation is removed by azeotropic distillation or in the flow of inert gas used.

## Patentansprüche

1. Verfahren zur Herstellung von d,l-α-Tocopherol durch die säurekatalysierte Kondensation von Trimethylhydrochinon mit Isophytol in Ethylen- oder Propylencarbonat oder einem Gemisch aus beiden Carbonaten oder in einem Gemisch aus einem oder beiden der Carbonate und einem nicht-polaren Lösungsmittel, wobei das Verfahren **dadurch gekennzeichnet ist, daß** die Kondensation ohne Verwendung einer Lewis-Säure in Gegenwart von höchstens 0,4 Gew.-%, bezogen auf das Gewicht von Isophytol, eines Säurekatalysators, ausgewählt aus Schwefelsäure, Methansulfonsäure, Ethansulfonsäure, Trifluormethansulfonsäure, p-Toluolsulfonsäure und Fluorsulfonsäure, durchgeführt wird.

2. Verfahren nach Anspruch 1, wobei der Katalysator Schwefelsäure oder p-Toluolsulfonsäure ist.

3. Verfahren nach Anspruch 1 oder 2, wobei die Menge an Säurekatalysator etwa 0,1 bis 0,4, vorzugsweise etwa 0,1 bis 0,2 Gew.-%, bezogen auf das Gewicht von Isophytol, beträgt.

4. Verfahren nach einem der Ansprüche 1 bis 3, wobei die Kondensation in Ethylencarbonat oder einem Gemisch davon mit einem nicht-polaren Lösungsmittel durchgeführt wird.

5. Verfahren nach einem der Ansprüche 1 bis 4, wobei das nicht-polare Lösungsmittel Hexan, Heptan oder Petrolether, vorzugsweise Heptan ist.

6. Verfahren nach einem der Ansprüche 1 bis 5, wobei das Volumenverhältnis des nicht-polaren Lösungsmittels zu dem Carbonat in dem Zweiphasen-Lösungsmittelsystem in dem Bereich von 0,3 : 1 bis 5 : 1 liegt.

7. Verfahren nach einem der Ansprüche 1 bis 6, wobei die Kondensation bei Temperaturen von etwa 50°C bis etwa 150 °C, vorzugsweise von etwa 80 °C bis etwa 120 °C, insbesondere bei etwa 100°C durchgeführt wird.

8. Verfahren nach einem der Ansprüche 1 bis 7, wobei Trimethylhydrochinon in einem molaren Überschuß gegenüber Isophytol von etwa 30 bis 65 %, vorzugsweise etwa 50 % verwendet wird.

9. Verfahren nach einem der Ansprüche 1 bis 8, wobei die Kondensation unter einer inerten Gasatmosphäre, vorzugsweise gasförmigem Stickstoff oder Argon, durchgeführt wird.

10. Verfahren nach einem der Ansprüche 1 bis 9, wobei etwa 10 bis 100 ml, vorzugsweise etwa 10 bis 50 ml, Carbonatlösungsmittel (Ethylen- und/oder Propylencarbonat) pro 100 mmol Trimethylhydrochinon verwendet werden, und wenn zusätzlich ein nicht-polares Lösungsmittel verwendet wird, werden etwa 50 bis 150 ml, vorzugsweise etwa 70 bis 120 ml, des nicht-polaren Lösungsmittels pro 100 mmol Isophytol verwendet.

11. Verfahren nach einem der Ansprüche 1 bis 10, wobei Isophytol oder eine Lösung aus Isophytol in dem eingesetzten nicht-polaren Lösungsmittel tropfenweise zu einer Lösung oder Suspension aus Trimethylhydrochinon und dem Säurekatalysator in Ethylen- oder Propylencarbonat oder einem Gemisch aus beiden Carbonaten zugegeben wird.

12. Verfahren nach einem der Ansprüche 1 bis 11, wobei das Wasser, das aus der Kondensation resultiert, durch azeotrope Destillation oder in dem Fluß des verwendeten Inertgases entfernt wird.

## Revendications

1. Procédé pour la fabrication de d,l-α-tocophérol par la condensation, catalysée par un acide, de triméthylhydroquinone avec de l'isophytol dans du carbonate d'éthylène ou de propylène ou un mélange des deux carbonates ou dans un mélange de l'un ou des deux carbonates et d'un solvant non polaire, lequel procédé est **caractérisé par** la mise en oeuvre de la condensation sans utilisation d'un acide de Lewis, en présence d'au plus 0,4 % en poids, par rapport au poids d'isophytol, d'un catalyseur acide choisi parmi l'acide sulfurique, l'acide méthanesulfonique, l'acide éthanesulfonique, l'acide trifluorométhanesulfonique, l'acide p-toluènesulfonique et l'acide fluorosulfonique.

2. Procédé selon la revendication 1, dans lequel le catalyseur est l'acide sulfurique ou l'acide p-toluènesulfonique.

3. Procédé selon la revendication 1 ou 2, dans lequel la quantité de catalyseur acide est d'environ 0,1 à 0,4, de préférence d'environ 0,1 à 0,2 % en poids, par rapport au poids d'isophytol.

4. Procédé selon l'une quelconque des revendications 1 à 3, dans lequel la condensation est mise en oeuvre dans du carbonate d'éthylène ou un mélange de celui-ci avec un solvant non polaire.

5. Procédé selon l'une quelconque des revendications 1 à 4, dans lequel le solvant non polaire est l'hexane, l'heptane ou l'éther de pétrole, de préférence l'heptane.

6. Procédé selon l'une quelconque des revendications 1 à 5, dans lequel le rapport en volume du solvant non polaire au carbonate dans le système solvant biphasique est situé dans la plage allant de 0,3/1 à 5/1.

7. Procédé selon l'une quelconque des revendications 1 à 6, dans lequel la condensation est effectuée à des températures d'environ 50°C à environ 150°C, de préférence d'environ 80°C à environ 120°C, en particulier à environ 100°C.

8. Procédé selon l'une quelconque des revendications 1 à 7, dans lequel on utilise la triméthylhydroquinone en un excès molaire par rapport à l'isophytol d'environ 30 à 65 %, de préférence d'environ 50 %.

9. Procédé selon l'une quelconque des revendications 1 à 8, dans lequel la condensation est mise en oeuvre sous une atmosphère de gaz inerte, de préférence de l'azote ou de l'argon gazeux.

10. Procédé selon l'une quelconque des revendications 1 à 9, dans lequel on utilise d'environ 10 à 100 ml, de préférence d'environ 10 à 50 ml de solvant carbonate (carbonate d'éthylène et/ou de propylène) pour 100 mmol de triméthylhydroquinone et, si on utilise de plus un solvant non polaire, on utilise d'environ 50 à 150 ml, de préférence d'environ 70 à 120 ml, de solvant non polaire pour 100 mmol d'isophytol.

11. Procédé selon l'une quelconque des revendications 1 à 10, dans lequel de l'isophytol ou une solution d'isophytol dans le solvant non polaire employé est ajouté goutte à goutte à une solution ou suspension de triméthylhydroquinone et de catalyseur acide dans un carbonate d'éthylène ou de propylène ou un mélange des deux carbonates.

12. Procédé selon l'une quelconque des revendications 1 à 11, dans lequel l'eau résultant de la condensation est éliminée par distillation azéotrope ou dans le courant de gaz inerte utilisé.
